# EUROPEAN PATENT APPLICATION

(11) **EP 2 392 354 A1**
(43) Date of publication of application: **07.12.2011**
(21) Application number: 10735909.3
(22) Date of filing: 29.01.2010
(51) Int. Cl.: A61K 47/20, A61K 9/10, A61K 9/14, A61K 9/20, A61K 31/075, A61K 45/00, A61K 47/26, A61K 47/30, A61K 47/32, A61K 47/34, A61K 47/38, A61P 1/16

(54) **FINELY PULVERIZED PHARMACEUTICAL COMPOSITION**

(30) Priority: 30.01.2009 JP 2009019771
(71) Applicant: Meiji Seika Pharma Co., Ltd., Tokyo 104-8002 (JP)
(72) Inventor: CHIKASE, Shigeru, Odawara-shi Kanagawa 250-0852 (JP); KAWADA, Dai, Yokohama-shi Kanagawa 222-8567 (JP)
(74) Representative: Gillard, Richard Edward
(86) International application number: PCT/JP2010/051262
(87) International publication number: WO 2010/087447

(57) **Abstract**

An objective of the present invention is to provide a composition that has improved oral absorption and comprises a slightly soluble drug, and to provide a process for producing the same. According to the present invention, there is provided a finely pulverized composition comprising a slightly soluble drug such as 22β-methoxyolean-12-ene-3β, 24(4β)-diol and a hydrophilic surfactant. According to the present invention, there is also provided a process for producing the finely pulverized composition according to the present invention, **characterized in that**, after the dispersion of a slightly soluble drug and a hydrophilic surfactant in a solvent, the dispersion is pulverized with a high-pressure homogenizer.

## Description

### TECHNICAL FIELD

The present invention relates to a finely pulverized composition comprising a slightly soluble drug and a hydrophilic surfactant. More specifically, the present invention relates to a finely pulverized composition comprising 22β-methoxyolean-12-ene-3β, 24(4(β)-diol, which is a slightly soluble drug, and a surfactant, and a finely pulverized composition comprising the above ingredients and additionally a pharmaceutically acceptable polymer compound.

### BACKGROUND ART

22β-Methoxyolean-12-ene-3,24(4β)-diol is a medicinal product synthesized using as a starting material soyasapogenol B contained in embryo axes of soybeans and is a pharmaceutical compound that has been developed mainly as therapeutic agents for liver diseases (see, for example, patent document 1).

This compound is less likely to be soluble in water or organic solvents and thus is a slightly soluble compound that cannot be expected to be absorbed into bodies of humans or the like without utilizing a special technique.

In order to improve oral absorption of slightly soluble drugs, an attempt to improve the absorbability has been made by amorphization, solid dispersion, or inclusion with an inclusion compound such as cyclodextrin or cholic acid (see, for example, non-patent documents 1 and 2). A method is also known in which the solubility is improved by mixing with a base material that causes self-emulsification.

Further, a method is disclosed in which a non-crosslinking surface modifier is added to a crystalline drug having low solubility and the mixture is ground using a grinding medium by mechanical means (see, for example, patent document 2). Furthermore, a method is disclosed in which fine particles are produced from a slightly soluble drug, polyvinyl pyrrolidone, and sodium dodecylsulfate by dry grinding using a grinding medium (see, for example, patent document 3).

These grinding methods, however, due to grinding of a drug using a grinding medium, pose problems of decomposition of the drug caused by the generation of heat or the occurrence of foreign matter caused by scraping of the medium used. Further, static electricity is generated during grinding. Accordingly, the ground drug is agglomerated, making it impossible to obtain a particulate drug having contemplated particle diameters. Thus, medicinal products cannot be produced by the above grinding methods.

### PRIOR ART DOCUMENTS

### Patent Documents

Patent document 1: Japanese Patent No. 3279574
Patent document 2: Japanese Patent No. 3602546
Patent document 3: Japanese Patent Application Laid-Open No. 99442/2004

### Non-patent Documents

Non-patent document 1: "Saikin no seizai gijutu to sono oyo (Current pharmaceutical technology and its application) I," published by Iyaku (Medicine and Drug) Journal Co. Ltd., p. 157-159, 1983
Non-patent document 2: "Saikin no seizai gijutu to sono oyo (Current pharmaceutical technology and its application) II," published by Iyaku (Medicine and Drug) Journal Co. Ltd., p. 158-162,1985

### SUMMARY OF THE INVENTION

There have been attempted to apply the above methods for improving oral absorption to the grinding of slightly soluble drugs such as 22β-methoxyolean-12-ene-3p, 24(4β)-diol. None of them have been satisfactory, for example, in the stability, solubility or ease of administration.

In the technical background, the present inventors have found that wet grinding of 22β-methoxyolean-12-ene-3β, 24(4β)-diol using a specific surfactant can realize the production of a highly physicochemically stable composition that does not cause agglomeration among particles during the production, comprises fine particles having a narrow particle size distribution and, at the same time, does not cause agglomeration among particles with an elapse of time. The present inventors have also found that a composition comprising fine particles, which do not cause agglomeration in a granulation step can be produced by additionally using a specific polymer compound. The present inventors have further found that these compositions exhibit a high level of elutability and a high level of oral absorbability. The present invention is based on such findings.

Accordingly, an object of the present invention is to provide a composition that has improved oral absorption and comprises a slightly soluble drug, and a process for producing the same.

According to the present invention, the following invention is provided.
(1) A composition comprising a slightly soluble drug and a hydrophilic surfactant.
(2) The composition according to (1), wherein the slightly soluble drug is a triterpene derivative or a pharmaceutically acceptable salt thereof.
(3) The composition according to (1), wherein the slightly soluble drug is 22β-methoxyolean-12-ene-3β, 24(β))-diol or a pharmaceutically acceptable salt thereof.
(4) The composition according to (1) or (2), wherein the hydrophilic surfactant is sodium laurylsulfate, a sucrose fatty acid ester, or polysorbate.
(5) The composition according to (1) or (2), which further comprises a polymer compound.
(6) The composition according to (5), wherein the polymer compound is a water soluble polymer.
(7) The composition according to (6), wherein the water soluble polymer is polyvinyl pyrrolidone or hypromellose.
(8) The composition according to (1) or (2), wherein the amount of the hydrophilic surfactant added is 10 to 400 mg per g of the triterpene derivative.
(9) The composition according to (5), wherein the amount of the polymer compound added is 30 to 800 mg per g of the triterpene derivative.
(10) The composition according to (3), wherein 22β-methoxyolean-12-ene-3β,24(4β)-diol has a cumulative 50% particle diameter of not more than 1.5 µm and a cumulative 90% particle diameter of not more than 3.0 µm.
(11) The composition according to (3), wherein 22β-methoxyolean-12-ene-3β,24(4β)-diol has a cumulative 50% particle diameter of not more than 1.0 µm and a cumulative 90% particle diameter of not more than 2.0 µm.
(12) A process for producing a composition according to any one of (1) to (3), characterized by comprising dispersing a slightly soluble drug and a hydrophilic surfactant in a solvent and then grinding the dispersion with a high-pressure homogenizer.
(13) A process for producing a composition according to (5), characterized by comprising dispersing a slightly soluble drug and a hydrophilic surfactant in a solvent, grinding the dispersion with a high-pressure homogenizer, and then further dissolving a polymer compound in the homogenate.
(14) The process according to (12) or (13), wherein the slightly soluble drug is 22β-methoxyolean-12-ene-3β, 24(4β)-diol or a pharmaceutically acceptable salt thereof.
(15) A composition comprising a slightly soluble drug and a hydrophilic surfactant, the composition being producible by a process according to any one of (12) to (14).

The composition of the present invention comprises a drug of homogeneous particles that are not agglomerated. The composition exhibits a high level of elutability within the digestive tract and has a high level of absorbability and thus can significantly enhance a blood level. Accordingly, the composition of the present invention can be expected to realize a high therapeutic effect of the slightly soluble drug.

### BRIEF DESCRIPTION OF THE DRAWINGS

[Fig. 1] Fig. 1 is a graph showing the results of a dissolution test for a bulk drug obtained by grinding with a high-pressure homogenizer (Example 1) and a bulk drug obtained by grinding with a jet mill (Comparative Example 1).
[Fig. 2] Fig. 2 is a graph showing the results of stability over time of cumulative 50% particle diameters of bulk drug particles in a suspension obtained by grinding at 5°C (□), 15°C (○), and 25°C (▲).
[Fig. 3] Fig. 3 is a graph showing the results of stability over time of cumulative 90% particle diameters of bulk drug particles in a suspension obtained by grinding at 5°C (□) 15°C (○), and 25°C (▲).
[Fig. 4] Fig. 4 is a graph showing the results of a dissolution test for fine granules of Example 5 and fine granules of Comparative Example 2.
[Fig. 5] Fig. 5 is a graph showing the results of a dissolution test of tablets produced using different excipients (Examples 6 to 8).
[Fig. 6] Fig. 6 is a graph showing the results of an oral absorption test with dogs for fine granules (•) of Example 4 produced by a bulk drug that has been ground with a high-pressure homogenizer, and fine granules (□) of Comparative Example 2.
[Fig. 7] Fig. 7 is a graph showing the results of an oral absorption test with dogs for fine granules (•) of Example 4 and tablets (△) of Example 6, that have been produced by a bulk drug that has been ground with a high-pressure homogenizer.

### DETAILED DESCRIPTION OF THE INVENTION

The slightly soluble drug used in the present invention refers to a drug that has low solubility in water, that is, a solubility of less than 0.1 mg/mL in water at 25°C. Slightly soluble drugs according to the present invention include triterpene derivatives described in Japanese Patent No. 3279574. Among them, 22β-methoxyolean-12-ene-3β,24(4β)-diol is preferred.

Triterpene derivatives may be represented by formula (I): 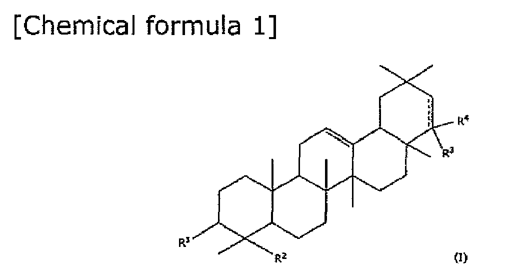 wherein
R¹ represents hydroxyl,
C₁₋₆ alkoxy,
C₁₋₆ alkylcarbonyloxy, or
optionally substituted aralkyloxy,
R² represents C₁₋₆ alkyl,
-CH₂OR⁵ wherein R⁵ represents a hydrogen atom, C₁₋₆ alkyl, optionally substituted aralkyl, or C₁₋₆ alkylcarbonyl,
formyl,
-COOR⁶ wherein R⁶ represents a hydrogen atom or C₁₋₆ alkyl, or
-CH₂N(R⁷)R⁸ wherein R⁷ and R⁸, which may be the same or different, represent a hydrogen atom or C₁₋₆ alkyl, or
R¹ and R² are combined with each other to represent -O-CR⁹(R¹⁰)-OCH₂- wherein R⁹ and R¹⁰, which may be the same or different, represent a hydrogen atom, C₁₋₆ alkyl, or aryl, and
R³ and R⁴, which may be the same or different, represent
a hydrogen atom,
hydroxyl,
C₁₋₆ alkyl,
hydroxy C₁₋₆ alkyl,
formyl,
-COOR¹¹ wherein R¹¹ represents a hydrogen atom or C₁₋₆ alkyl, or
-OR¹² wherein R¹² represents C₁₋₆ alkyl, optionally substituted aralkyl, C₁₋₆ alkylcarbonyl, optionally substituted arylcarbonyl, C₂₋₆ alkenyl, C₂₋₆ alkenylcarbonyl, or optionally substituted aryl alkenylcarbonyl, or
R³ and R⁴ together represent methylene,
--- in the formula represents a single bond or a double bond, provided that, when --- represents a double bond, R⁴ is absent.

In the present specification, the term "alkyl" as a group or a part of a group means any of a straight chain type or a branched chain type. Specific examples thereof include methyl, ethyl, propyl, iso-propyl, n-butyl, iso-butyl, and t-butyl. The term "halogen atom" means a fluorine, chlorine, bromine, or iodine atom. The term "aryl" as a group or a part of a group preferably means phenyl, naphthyl, tolyl, methoxyphenyl or the like. The term "aralkyl" as a group or a part of a group preferably means phenyl C₁₋₄ alkyl, more preferably benzyl, phenethyl or the like. One or more hydrogen atoms, preferably one or two hydrogen atoms on "aryl" or "aralkyl" are optionally substituted, and specific examples of substituents include hydroxyl, C₁₋₆ alkoxy (preferably C₁₋₄ alkoxy, more preferably methoxy), halogen atoms, amino, dimethylamino, acetoxy, methylenedioxy, and nitro. Examples of substituted groups include methoxyphenyl, hydroxyphenyl, dihydroxyphenyl, and dimethoxyphenyl.

In formula (I), C₁₋₆ alkoxy represented by R¹ is preferably C₁₋₄ alkoxy, more preferably methoxy or ethoxy. Specific examples thereof include methoxy, ethoxy, propyloxy, butyloxy, pentyloxy, and hexyloxy. C₁₋₆ alkylcarbonyl is preferably C₁₋₄ alkylcarbonyl, and specific examples thereof include acetyl, propionyl, butyryl, pentanoyl, and hexanoyl. Examples of preferred aralkyloxy represented by R¹ include benzyloxy, phenethyloxy, methylbenzyloxy, and naphthylmethyloxy.
In formula (I), -CH₂OR⁵ represented by R² is preferably -CH₂OH, -CH₂O-C₁₋₄ alkyl, -CH₂O-(phenylC₁₋₄ alkyl), or -CH₂O-CO-C₁₋₄ alkyl, more preferably hydroxymethyl. -COOR⁶ represented by R² is preferably -COO-C₁₋₆ alkyl and COOH.

In formula (I), R¹ and R² may be combined with each other to form -O-CR⁹(R¹⁰)-OCH₂-. Here R⁹ and R¹⁰, which may be the same or different, represent a hydrogen atom, C₁₋₆ alkyl, or aryl. Preferred examples thereof include the case where both R⁹ and R¹⁰ represent C₁₋₆ alkyl, preferably C₁₋₄ alkyl, more preferably methyl or ethyl and the case where any one of R⁹ and R¹⁰ represents a hydrogen atom while the other represents aryl, preferably phenyl, tolyl, xylyl, biphenyl, naphthyl, anthryl, or phenanthryl.

In formula (I), C₁₋₆ alkyl represented by R³ or R⁴ is preferably C₁₋₄ alkyl, more preferably methyl or ethyl. Hydroxy C₁₋₆ alkyl represented by R³ and R⁴ is preferably hydroxy C₁₋₄ alkyl, more preferably hydroxymethyl.

In formula (I), -COOR¹¹ represented by R³ or R⁴ preferably represents -COOH or -COO-C₁₋₄ alkyl.

Further, in formula (I), R¹² in -OR¹² represented by R³ or R⁴ represents C₁₋₆ alkyl, aralkyl, C₁₋₆ alkylcarbonyl, arylcarbonyl, C₂₋₆ alkenyl, C₂₋₆ alkenylcarbonyl, or aryl alkenylcarbonyl. Here C₂₋₆ alkenyl is preferably C₂₋₄ alkenyl, and specific examples thereof include vinyl, propenyl, allyl, butenyl, 2-methylpropenyl, pentenyl, and hexenyl. Examples of aralkyl include benzyl, phenethyl, methyl benzyl, naphthylmethyl, and phenylpropyl. C₁₋₆ alkylcarbonyl is preferably C₁₋₄ alkylcarbonyl, and specific examples thereof include acetyl, propionyl, butyryl, pentanoyl, and hexanoyl. Examples of preferred arylcarbonyl include benzoyl and naphthylcarbonyl. C₂₋₆ alkenyl is preferably C₂₋₄ alkenylcarbonyl, and specific examples thereof include acryloyl, allylcarbonyl, and butenoyl. C₂₋₆ alkenylcarbonyl is preferably C₂₋₄ alkenylcarbonyl. Specific examples of aryl alkenylcarbonyl include cinnamoyl and phenylbutenoyl.

R³ and R⁴ together represent methylene.

In formula (I), --- represents a single bond or a double bond.

When in the formula represents a double bond, preferably, R¹ represents a hydrogen atom, R² represents -CH₂OH, or R¹ and R² are combined with each other to represent -O-CR⁹(R¹⁰)-OCH₂- wherein R⁹ and R¹⁰ are as defined above, and R³ represents a hydrogen atom.

In compounds of formula (I), various isomers exist. The present invention embraces both the isomers and mixtures thereof. The presence of isomers derived from other groups in formula (I) is also considered. These isomers and mixtures thereof are also embraced in formula (I).

Compounds of formula (I) preferably have a configuration represented by formula (I-1):

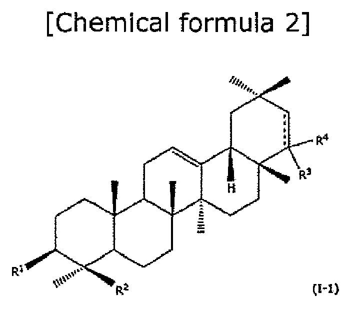

The triterpene derivatives may be used in the form of a pharmaceutically acceptable salt in the composition according to the present invention. The salts of the triterpene derivatives can easily be formed by allowing a pharmaceutically acceptable base to act on triterpene derivatives. Preferred bases include inorganic bases such as sodium hydroxide, potassium hydroxide, aluminum hydroxide, sodium carbonate, potassium carbonate, and sodium hydrogencarbonate, and organic bases such as piperazine, morpholine, piperidine, ethylamine, and trimethylamine.

Methods for grinding drugs are classified roughly into two methods, i.e., dry type and wet type methods according to a state during grinding. During grinding, static electricity is generated due to friction among the particles. In particular, in dry grinding, the generation of static electricity causes adherence of particles onto a grinder or the like or secondary agglomeration in which ground particles are again agglomerated. Consequently, the particle size of ground bulk drug particles is increased, and, thus, particle diameters of predetermined drug particles cannot be obtained. Agglomeration further proceeds during the storage of the ground drug, and, thus, dry grinding is not a preferable method. An increase in particle size refers to a lowering in absorbability of the drug in digestive tracts, making it impossible to maintain a given quality level as products. Further, high susceptibility to agglomeration leads to adherence of particles onto a production apparatus and the like, resulting in lowered yield or poor handling during the production. Accordingly, that the particle size remains unchanged over time even when the ground drug is stored is a pharmaceutically important factor.

On the other hand, the wet grinding is free from a problem involved in the dry grinding and thus is suitable as a grinding method used in the present invention. Since, however, also in the wet grinding, ground particles are agglomerated in a solution, the wet grinding is not a complete method that does not cause agglomeration.

As described in Examples below, it has been found that the use of a specific surfactant in the wet grinding can allow drug particles to be physiochemical stably maintained without causing agglomeration. That is, when a specific surfactant is added as an additive, even the wet grinding can produce particles having desired particle diameters that do not cause agglomeration.

Hydrophilic surfactants are desired as the surfactant used in the present invention. Among them, sodium laurylsulfate, sucrose fatty acid esters, polysorbate and the like are preferred. Sodium laurylsulfate is more preferred when the dispersibility in water and physiochemical stability over time of the bulk drug used in the present invention are taken into consideration.

That is, the composition according to one aspect of the present invention is a suspension comprising a slightly soluble drug and the surfactant.

The addition amount of the surfactant used in the present invention may be 10 to 400 mg, preferably 50 to 200 mg, more preferably 50 to 100 mg, per g of the drug used in the present invention, from the viewpoints of dispersion of the drug in water and the viscosity and fluidity of the suspension.

Regarding the concentration of the composition according to the present invention, the solid content may be properly regulated depending upon the situation. The concentration may be 5 to 50%, preferably 25 to 40%, in terms of the solid content of the bulk drug in the suspension, from the viewpoints of smooth practice of the wet grinding step and yield.

Grinding machines usable in the present invention include a high-pressure homogenizer that allows pulverizing to be performed through the action of shear force or cavitation by applying high pressure to a suspension when the bulk drug is passed through fine slits while circulating the suspension, wet type bead mills in which beads having various materials and sizes are mixed into a suspension followed by stirring to perform pulverizing by collision of beads against each other and the like, and ultraprecision grinding machines that perform pulverizing through the action of high-speed shear force generated by supplying a suspension into a portion of very narrow clearance between two disks being rotated at a high speed.

The high-pressure homogenizer is best suited as the grinding machine used in the present invention because foreign matter is not formed. The type of the high-pressure homogenizer is not particularly limited. Grinding conditions for the high-pressure homogenizer may be properly regulated depending upon the amount of the drug ground. The treatment pressure of the high-pressure homogenizer is preferably 150 to 200 MPa, more preferably 170 to 200 MPa, particularly preferably 190 to 200 MPa. The number of times of treatment is preferably 20 to 40, more preferably 25 to 30.

The composition according to the present invention may be produced by the following process. Specifically, the composition according to the present invention may be produced by first dissolving a surfactant in a solvent such as water, adding a drug into the solution, dispersing the drug with a conventional stirrer, and grinding the dispersion solution with a high-pressure homogenizer.

Preferably, the particles of the drug contained in the composition according to the present invention have a cumulative 50% particle diameter of not more than 1.5 µm and a cumulative 90% particle diameter of not more than 3.0 µm. More preferably, the cumulative 50% particle diameter and the cumulative 90% particle diameter are not more than 1.0 µm and not more than 2.0 µm, respectively. In the present invention, when a cumulative curve is determined by presuming the total volume of a powder population, of which the particle size distribution is analyzed, to be 100%, the particle diameter of a point at which the cumulative curve is 50% and the particle diameter of a point at which the cumulative curve is 90% are "cumulative 50% particle diameter" and "cumulative 90% particle diameter" (µm), respectively.

The diameters of drug particles obtained by the grinding can be measured by a laser diffraction/scattering-type particle size distribution measuring machine. Specifically, for example, Microtruck (tradename) sold by NIKKISO Co., Ltd. is preferred.

One of features of the composition of the present invention is that agglomeration does not occur during the production of the composition. Here the expression "agglomeration does not occur" means that drug particles are not precipitated in the suspension and, even though drug particles are precipitated, after redispersion of the precipitate, the particle diameter after the wet grinding is maintained and that, in a powder form, the particle diameter of the drug obtained by drying the suspension is the same as the particle diameter after the wet grinding.

When the grinding step is transferred to a granulation step, the suspension as such is sometimes stored for a given period of time. Even in this case, the composition and suspension according to the present invention do not cause agglomeration at a storage temperature of 5 to 25°C and maintains a physicochemically stable state.

A composition of the present invention or a suspension of the present invention that further comprises a polymer compound may be mentioned as a composition according to another aspect of the present invention. The composition and the suspension can be provided in granules.

Granules according to the present invention can be produced from the suspension obtained in the present invention. Drug granules can be obtained by granulating or drying the suspension. However, when the suspension as such is granulated or dried, drug particles in the suspension are agglomerated during granulation/drying and, consequently, the particle diameters are much larger than the contemplated particle diameter. Accordingly, the wet grinding does not make sense. Therefore, preferably, the granules according to the present invention are produced so that agglomeration does not occur during granulation/drying.

A method effective in avoiding the agglomeration is to add a specific polymer compound to the suspension according to the present invention before the granulation. According to this method, the ground drug particles are not agglomerated, and the particle diameter of the drug can be kept constant. Preferably, the polymer compound is dissolved in and mixed into the suspension according to the present invention at a stage before the granulation/drying.

Water soluble polymers are preferred as the polymer compound used in the particles according to the present invention. Among them, polyvinyl polymers and cellulosic polymers, for example, polyvinyl pyrrolidone and hypromellose are preferred.

The addition amount of the polymer compound used in the present invention is 30 to 800 mg, preferably 30 to 200 mg, more preferably 50 to 100 mg, per g of the bulk drug of the present invention.

Orally administrable pharmaceutical preparations such as powders, fine subtilaes, granules, tablets, capsules, suspensions, and liquids can be produced by further incorporating a pharmaceutically acceptable carrier in the composition of the present invention. These pharmaceutical preparations can be produced by conventional methods.

Additives for medicinal products used in pharmaceutical preparations may be used as the pharmaceutically acceptable carrier. Specific examples thereof include excipients, disintegrators, binders, dispersants, lubricants, coating agents, and plasticizers. If necessary, sweetening agents, preservatives, colorants, flavoring agents and the like may be properly incorporated.

Excipients include lactose, saccharose, D-mannitol, starch, crystalline cellulose, and partially gelatinized starch.

Disintegrators include croscarmellose sodium, carboxymethyl starch sodium, carboxymethylcelulose, low substituted hydroxypropylcellu lose, and crospovidone.

Binders include hydroxypropylcellulose, hydroxypropylmethylcellulose, hypromellose, and polyvinyl pyrrolidon. Dispersants include light anhydrous silicic acid, carmellose sodium, and xanthan gum.

Lubricants include magnesium stearate, calcium stearate, and talc.

Coating agents include hydroxypropylcellulose, hydroxypropylmethylcellulose, hydroxypropylmethylcellulose acetate succinate, hydroxypropylmethylcellulose phthalate, cellulose acetate phthalate, ethylcellulose, water dispersion of ethylcellulose, dispersion of ethyl acrylate-methyl methacrylate copolymer, and methacrylic acid copolymer LD.

Plasticizers include triacetin, stearic acid, polyethylene glycol, and triethyl citrate.

Sweetening agents include aspartame, xylitol, saccharine sodium, glycyrrhizinic acid, and stevia extracts.

Preservatives include p-oxybenzoic acid esters, chlorobutanol, and benzyl alcohol.

Colorants include riboflavins or yellow iron sesquioxide, red iron sesquioxide, food dyes such as food yellow No. 5 and food blue No.2, and food lake dyes.

Flavoring agents include natural flavoring agents such as fruit flavoring agents, fruit skin flavoring agents, bark flavoring agents, seed flavoring agents, foliage flavoring agents, and flower flavoring agents, and synthetic flavoring agents such as citral, citronellal, citronellol, cis-jasmine, and cis-3-hexanol.

The dose of the composition according to the present invention may vary depending, for example, upon the age, weight, conditions, and severity of condition of patients. However, when the slightly soluble drug is a triterpene derivative, the composition of the present invention may be usually orally administered at a dose of 25 to 800 mg (active ingredient) per adult per day.

The composition according to the present invention may be used as a pharmaceutical composition for the treatment and prevention of adaptation diseases of slightly soluble drugs. When the active ingredient of the composition according to the present invention is a triterpene derivative, the composition according to the present invention may be used as therapeutic and prophylactic agents for liver diseases and hyperferremia. Liver diseases include acute and chronic viral hepatitis, autoimmune hepatitis, and drug-induced, addictive, alcohol-induced, intrahepatic cholestatic, and congenital metabolic disorder hepatopatlly. Here "hepatitis" and "hepatopathy" mean hepatic inflammatory diseases and are used as a concept embracing fatty liver, hepatic cirrhosis, and hepatocarcinoma depending on progression of symptoms.

### EXAMPLES

The present invention is further illustrated by the following Examples and Comparative Examples that are not intended as a limitation of the invention.

### Example 1: Grinding of bulk drug

An aqueous solution obtained by dispersing 3.0 kg of 22β-methoxyolean-12-ene-3β,24(4β)-diol bulk drug (manufacturer: Meiji Seika Kaisha Co., Ltd., cumulative 50% particle diameter: about 50 µm, cumulative 90% particle diameter: about 100 µm), 0.3 kg of sodium laurylsulfate, and 6.7 kg of purified water at a weight ratio of 30 : 3 : 67 was ground with a high-pressure homogenizer (model: Microfluidizer, manufacturer: Microfluidics) under grinding conditions (treatment pressure and number of times of treatment) shown in Table 1 to give a suspension. Regarding the particle size of the bulk drug in the suspension, the cumulative 50% particle diameter and the cumulative 90% particle diameter were measured with a particle size distribution measuring device (manufacturer: NIKISSO Co., Ltd., model: Microtruck ×100). The results are shown in Table 1.

**[Table 1]**

| Grinding conditions | Treatment pressure (MPa) | Number of times of treatment (times) | Cumulative 50% particle diameter (µm) | Cumulative 90% particle diameter (µm) |
|---|---|---|---|---|
| 1 | 150 | 40 | 1.4 | 2.6 |
| 2 | 200 | 40 | 1.0 | 1.8 |
| 3 | 200 | 50 | 0.8 | 1.6 |
| 4 | 200 | 80 | 0.6 | 1.2 |

### Comparative Example 1: Grinding of bulk drug

22β-Methoxyolean-12-ene-3β, 24(4β)-diol bulk drug (manufacturer: Meiji Seika Kaisha Co., Ltd., cumulative 50% particle diameter: about 50 µm, cumulative 90% particle diameter: about 100 µm) (14 kg) was ground with a jet mill (manufacturer: Nippon Pneumatic Mfg. Co., Ltd., model: Jet Mill PJM-100) under conditions of 0.54 MPa. Regarding the particle diameter of the ground bulk drug, the cumulative 50% particle diameter was measured with a particle size distribution measuring device (manufacturer: NIKKISO Co., Ltd., model: Mircotruck X100) for a sample obtained by dispersing the ground bulk drug in a 0.01% aqueous sodium laurylsulfate solution. As a result, it was found that the cumulative 50% particle diameter of the ground bulk drug was 2.5 µm.

### Example 2: Dissolution test (1)

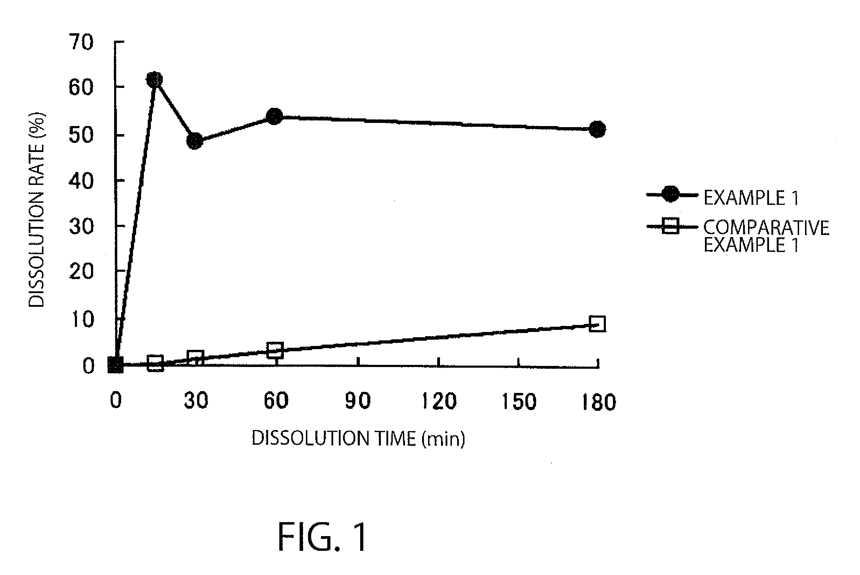

A dissolution test was carried out for the suspension produced in Example 1 (grinding conditions 3) and a liquid obtained by suspending the ground bulk drug obtained in Comparative Example 1 in water.
Each of the suspensions in an amount corresponding to 50 mg of the 22β-methoxyolean-12-ene-3β,24(4β)-diol bulk drug was accurately weighed, and a test was carried out by a dissolution test method, second method (paddle method), specified in Japanese Pharmacopoeia, at a rotation speed of 100 rpm (rotation speed of paddle) using 900 mL of a 1% aqueous polysorbate 80 solution as a test solution. After 15 min, 30 min, 60 min, and 180 min from the start of dissolution, 5 mL of the test solution was collected and was filtered by a membrane filter having a pore diameter of 0.2 µm to give measurement samples. The samples were analyzed by high-performance liquid chromatography (manufacturer: Shimadzu Seisakusho Ltd., model:LC-10ATvp) to determine dissolution rate for each sample.
The results are shown in Fig. 1. For the 22β-methoxyolean-12-ene-3β,24(4β)-diol bulk drug of Example 1 (grinding conditions 3), the dissolution rate exceeded 60% in 15 min.
On the other hand, the dissolution rate for Comparative Example 1 was low and 10% three hr after the start of the dissolution.
The above results demonstrated that the bulk drug ground by a high-pressure homogenizer had significantly improved elutability.

### Example 3: Stability over time of ground bulk drug in suspension

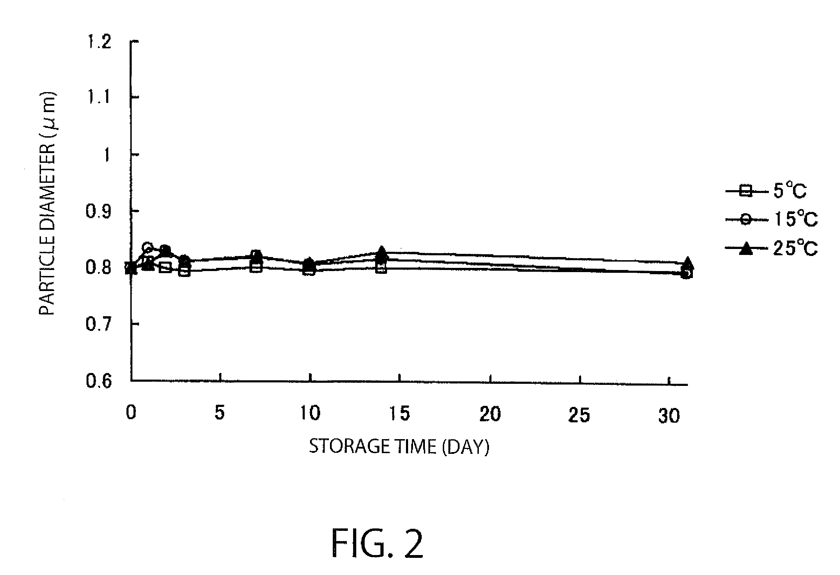

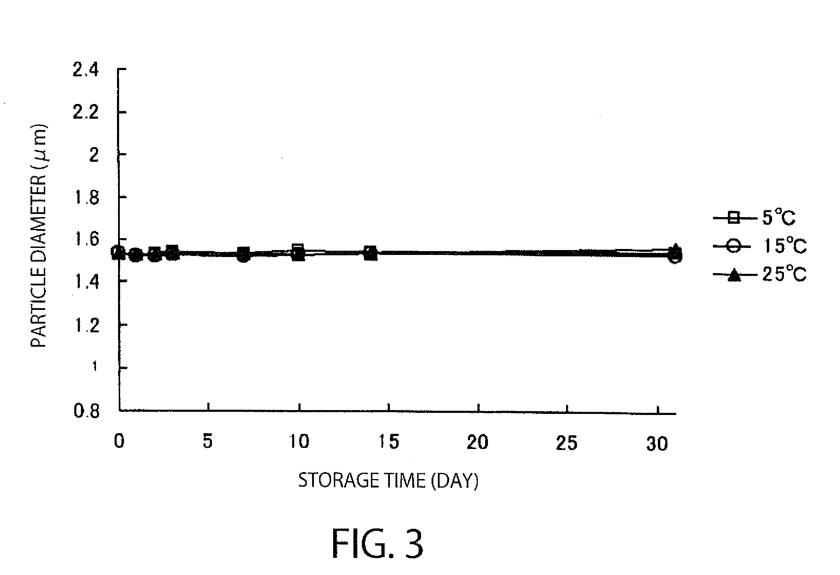

The suspension obtained by grinding in Example 1 (grinding conditions 3) was stored in a standing state to confirm whether the particle size of bulk drug particles in the suspension undergoes a change over time. The suspension was stored at 5°C, 15°C, and 25°C in a static state.
The cumulative 50% particle diameter and the cumulative 90% particle diameter were measured with an identical particle size distribution measuring device over time. The results are shown in Figs. 2 and 3. Both the cumulative 50% particle diameter and the cumulative 90% particle diameter remained unchanged for one month, indicating that secondary agglomeration did not occur. That is, it was confirmed that grinding by the method proposed by the present application can allow sodium laurylsulfate in the suspension to suppress secondary agglomeration, whereby physicochemically stable bulk drug particles can be obtained.

### Example 4: Production of fine granules (1)

A 4.3% aqueous hypromellose (also known as hydroxypropylmethylcellulose, tradename: TC-5R, manufacturer: The Shin-Etsu Chemical Co., Ltd.) solution (7.6 L) was added to and mixed with 7.3 L of the suspension obtained in Example 1 (grinding conditions 2). The mixed liquid was sprayed with a fluidized bed granulator (model: SPIR-A-FLOW SFC-15, manufacturer: Freund Corporation) onto a mixture of 250 g of croscarmellose sodium with 7250 g of D-mannitol to obtain granules. The granules were subjected to particle size reduction with a screening mill (model: COMIL, manufacturer: QUADRO) to produce fine granules.

### Example 5: Production of fine granules(2)

A 4.3% aqueous polyvinyl pyrrolidone (grade: K30, manufacturer: BASF) solution (7.6 L) was added to and mixed with 7.3 L of the suspension obtained in Example 1 (grinding conditions 2). The mixed liquid was sprayed with a fluidized bed granulator (model: SPIR-A-FLOW SFC-15, manufacturer: Freund Corporation) onto a mixture of 250 g of croscarmellose sodium with 7250 g of D-mannitol to obtain granules. The granules were subjected to particle size reduction with a screening mill (model: COMIL, manufacturer: QUADRO) to produce fine granules.

### Comparative Example 2: Production of fine granules

The 22β-methoxyolean-12-ene-3β,24(4β)-diol bulk drug that has a cumulative 50% particle diameter of 2.5 µm and has been obtained by grinding with a jet mill in Comparative Example 1 (25.0 mg), 8.75 mg of croscarmellose sodium, 2.5 mg of hydroxypropylcellulose, and 83.75 mg of D-mannitol were mixed together in a granulator (manufacturer: Fukae Kogyo Co., Ltd., model: High-speed mixer LFS-GS-2J), and wet granulation was carried out while spraying 130 mL of purified water, followed by drying. The dried product was ground with a cutting mill (manufacturer: DALTON CORPORATION, model: Power Mill P-02S) to produce fine granules.

### Comparative Example 3: Production of fine granules

22β-Methoxyolean-12-ene-3β,24(4β)-diol bulk drug (2.2 kg), 0.22 kg of sodium laurylsulfate, and 4.9 kg of purified water were dispersed at a weight ratio of 30 : 3 : 67 to prepare 7.3 L of a suspension. The suspension was subjected to wet grinding with a high-pressure homogenizer used in Example 1. The grinding was carried out under conditions of a treatment pressure of 200 MPa and a number of times of treatment of 40. After the grinding, the cumulative 50% particle diameter of the bulk drug was measured with the particle size distribution measuring device used in Example 1 and was found to be 1.0 µm.
The wet ground suspension as such was sprayed, without the addition of a water soluble polymer, onto a mixture of 250 g of croscarmellose sodium with 7250 g of D-mannitol to obtain granules. The granules were subjected to particle size reduction with a screening mill (model: COMIL, manufacturer: QUADRO) to produce fine granules.

### Comparative Example 4: Production of fine granules

22β-Methoxyolean-12-ene-3β,24(4β)-diol bulk drug (2.2 kg), 0.22 kg of sodium laurylsulfate, and 4.9 kg of purified water were dispersed at a weight ratio of 30 : 3 : 67 to prepare 7.3 L of a suspension. The suspension was subjected to wet grinding with a high-pressure homogenizer used in Example 1. The grinding was carried out under conditions of a treatment pressure of 200 MPa and a number of times of treatment of 40. After the grinding, the cumulative 50% particle diameter of the bulk drug was measured with the particle size distribution measuring device used in Example 1 and was found to be 1.0 µm.

A 4.3% aqueous low substituted hydroxypropylcellulose (grade: LH-21, manufacturer: The Shin-Etsu Chemical Co., Ltd.) solution (7.6 L) was added to and mixed with the wet ground suspension. The mixed liquid was sprayed with a fluidized bed granulator (model: SPIR-A-FLOW SFC-15, manufacturer: Freund Corporation) onto a mixture of 250 g of croscarmellose sodium with 7250 g of D-mannitol to obtain granules. The granules were subjected to particle size reduction with a screening mill (model: COMIL, manufacturer: QUADRO) to produce fine granules.

### Comparative Example 5: Production of fine granules

22β-Methoxyolean-12-ene-3β, 24(4β)-diol bulk drug (2.2 kg), 0.22 kg of sodium laurylsulfate, and 4.9 kg of purified water were dispersed at a weight ratio of 30 : 3 : 67 to prepare 7.3 L of a suspension. The suspension was subjected to wet grinding with a high-pressure homogenizer used in Example 1. The grinding was carried out under conditions of a treatment pressure of 200 MPa and a number of times of treatment of 40. After the grinding, the cumulative 50% particle diameter of the bulk drug was measured with the particle size distribution measuring device used in Example 1 and was found to be 1.0 µm.
A 4.3% aqueous Macrogol (grade: 6000, manufacturer: Sanyo Chemical Industries, Ltd.) solution (7.6 L) was added to and mixed with the wet ground suspension. The mixed liquid was sprayed with a fluidized bed granulator (model: SPIR-A-FLOW SFC-15, manufacturer: Freund Corporation), onto a mixture of 250 g of croscarmellose sodium with 7250 g of D-mannitol to obtain granules. The granules were subjected to particle size reduction with a screening mill (model: COMIL, manufacturer: QUADRO) to produce fine granules.

### Example 6: Production of tablets (1)

A 10% aqueous hypromellose (also known as hydroxypropylmethylcellulose, tradename: TC-5R, manufacturer: The Shin-Etsu Chemical Co., Ltd.) solution (225 mL) was added to and mixed with 428.6 g of the suspension obtained in Example 1. The mixed liquid was sprayed with a fluidized bed granulator (model: Multiplex Granulator MP-01, manufacturer: POWREX CORPORATION) onto a mixture of 540 g of lactose with 19.5 g of croscarmellose sodium to obtain granules.
Croscarmellose sodium (20 g) and 4 g of light anhydrous silicic acid were added to 124.5 g of the granules. Further, 1.5 g of magnesium stearate was added to and mixed with the mixture to obtain granules for tableting. The granules for tableting were tabletted at a pressure of 550 kg to 580 kg to produce tablets of 8 mmϕ.

### Example 7: Production of tablets (2)

A 10% aqueous hypromellose (also known as hydroxypropylmethylcellulose, tradename: TC-5R, manufacturer: The Shin-Etsu Chemical Co., Ltd.) solution (225 mL) was added to and mixed with 428.6 g of the suspension obtained in Example 1. The mixed liquid was sprayed with a fluidized bed granulator (model: Multiplex Granulator MP-01, manufacturer: POWREX CORPORATION) onto a mixture of 540 g of D-mannitol with 19.5 g of croscarmellose sodium to obtain granules.
Croscarmellose sodium (20 g) and 4 g of light anhydrous silicic acid were added to 124.5 g of the granules. Further, 1.5 g of magnesium stearate was added to and mixed with the mixture to obtain granules for tableting. The granules for tableting were tabletted at a pressure of 550 kg to 580 kg to produce tablets of 8 mmϕ.

### Example 8: Production of tablets (3)

A 10% aqueous hypromellose (also known as hydroxypropylmethylcellulose, tradename: TC-5R, manufacturer: The Shin-Etsu Chemical Co., Ltd.) solution (225 mL) was added to and mixed with 428.6 g of the suspension obtained in Example 1. The mixed liquid was sprayed with a fluidized bed granulation drier (model: Multiplex Granulator MP-01, manufacturer: POWREX CORPORATION) onto a mixture of 540 g of crystalline cellulose (Ceolus KG-802) with 19.5 g of croscarmellose sodium to obtain granules.
Croscarmellose sodium (20 g) and 4 g of light anhydrous silicic acid were added to 124.5 g of the granules. Further, 1.5 g of magnesium stearate was added to and mixed with the mixture to obtain granules for tableting. The granules for tableting were tabletted at a pressure of 550 kg to 580 kg to produce tablets of 8 mmϕ.

### Example 9: Measurement of cumulative 50% particle diameter of bulk drug particles

The cumulative 50% particle diameter of bulk drug particles in the fine granules in Examples 4 and 5 and Comparative Examples 3 to 5 was measured with a particle size distribution measuring device (manufacturer: NIKKISO Co., Ltd., model: Mircotruck X100). The results are shown in Table 2. It was confirmed that, for the water soluble polymer used in Examples 4 and 5, the bulk drug particles did not cause agglomeration. On the other hand, it was confirmed that, for the water soluble polymer used in Comparative Examples 3 to 5, the particle diameter of the bulk drug particles was clearly increased.

**[Table 2]**

| | Type of water soluble polymer | Cumulative 50% particle diameter (µm) |
|---|---|---|
| Example 4 | Hypromellose | 0.9 |
| Example 5 | Polyvinyl pyrrolidone | 0.9 |
| Comparative Example 3 | Not added | 13.9 |
| Comparative Example 4 | L-HPC | 22.6 |
| Comparative Example 5 | Macrogol 6000 | 31.4 |

### Example 10: Dissolution test (2)

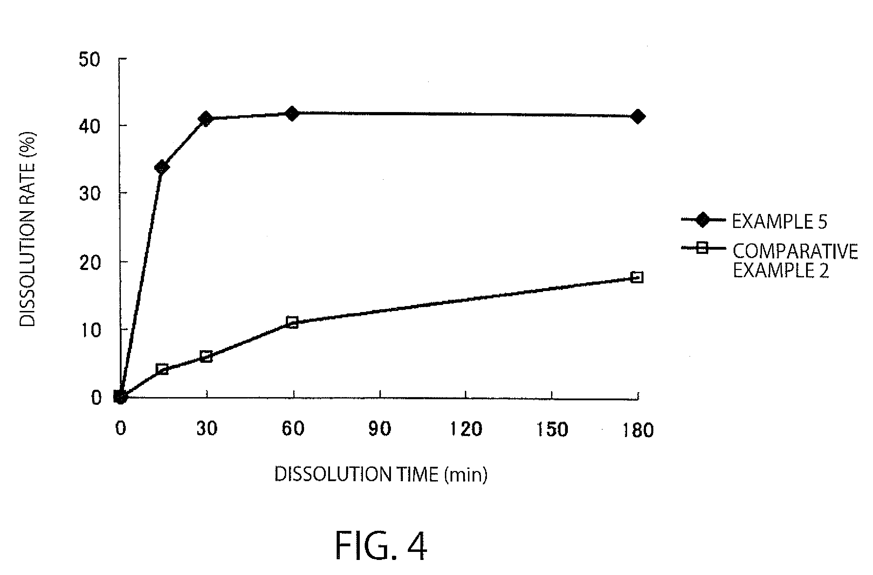

A dissolution test was carried out for the fine granules of Example 5 and the fine granules of Comparative Example 2. Each of the granules in an amount corresponding to 50 mg of the 22β-methoxyolean-12-ene-3β,24(4β)-diol bulk drug was accurately weighed, and a test was carried out by a dissolution test method, second method, at a rotation speed of 100 rpm using 900 mL of a 1% aqueous polysorbate 80 solution as a test solution. After 15 min, 30 min, 60 min, 120 min, and 180 min from the start of dissolution, 5 mL of the test solution was collected and was filtered by a membrane filter having a pore diameter of 0.2 µm to give measurement samples. The samples were analyzed by high-performance liquid chromatography (manufacturer: Shimadzu Seisakusho Ltd., model: LC-10ATvp) to determine dissolution rate for each sample.
The results are shown in Fig. 4. The dissolution rate of 60 min after the start of the dissolution of the pharmaceutical preparation of Example 5 produced using the bulk drug ground by a high-pressure homogenizer was 4 times higher than that of the pharmaceutical preparation of Comparative Example 2 produced using the bulk drug ground by a jet mill.

### Example 11: Dissolution test (3)

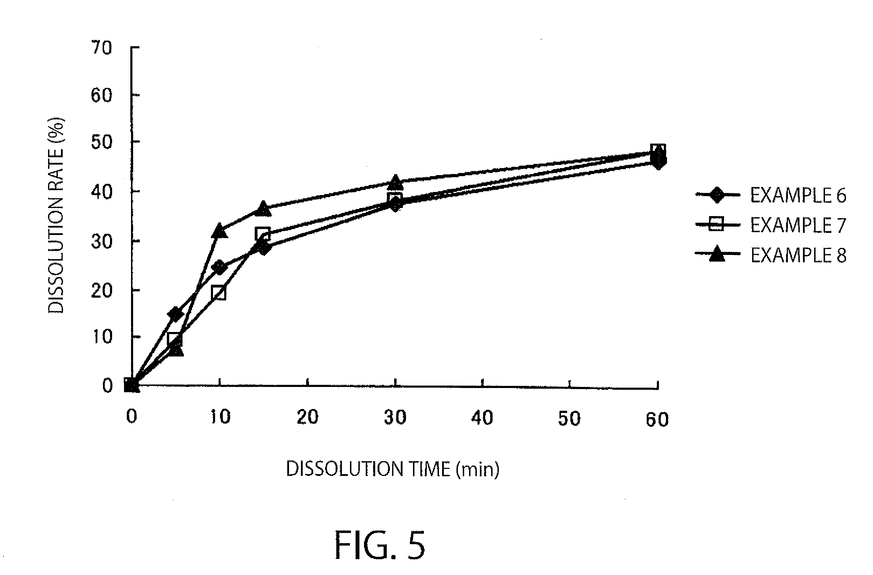

A dissolution test was carried out for tablets that were produced in Examples 6 to 8 and contained the 22β-methoxyolean-12-ene-3β, 24(4β)-diol bulk drug (25 mg). The test was carried out by a dissolution test method, second method (paddle method), specified in Japanese Pharmacopoeia at a rotation speed of 50 rpm (rotation speed of paddle) using 900 mL of a 1% aqueous polysorbate 80 solution as a test solution. After 5 min, 10 min, 15 min, 30 min, and 60 min from the start of dissolution, 5 mL of the test solution was collected and was filtered by a membrane filter having a pore diameter of 0.2 µm to give measurement samples. The samples were analyzed by high-performance liquid chromatography (manufacturer: Shimadzu Seisakusho Ltd., model: LC-10ATvp) to determine dissolution rate for each sample. The results are shown in Fig. 5. All the tablets had good dissolution.

### Example 12: Oral absorption test in dogs (1)

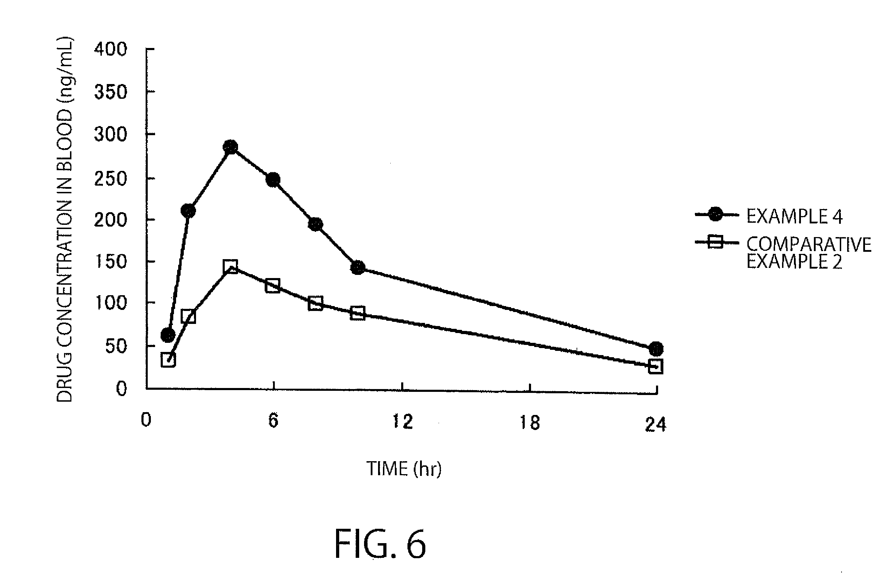

Oral absorbability in dogs (male beagle dogs, weight about 11 kg) was evaluated for the fine granules of Example 4 and the fine granules of Comparative Example 2. The test was carried out by a crossover test using 10 dogs. Under Fed state, both the pharmaceutical preparations in an amount corresponding to 50 mg of 22β-methoxyolean-12-ene-3β, 24(4β)-diol were taken with 10 mL of water. After the elapse of predetermined times (1 hr, 2 hr, 4 hr, 6 hr, 8 hr, 10 hr, and 24 hr), blood was collected, and the drug concentration in blood was measured with LC-MS/MS.
The results are shown in Fig. 6. For the fine granules of Example 4 according to the present invention, as compared with the fine granules of Comparative Example 2, both the maximum drug concentration in blood (Cmax) and the area under the time curve for drug concentration in blood (AUC) were increased, indicating that the oral absorbability was significantly improved.

### Example 13: Oral absorption test in dogs (2)

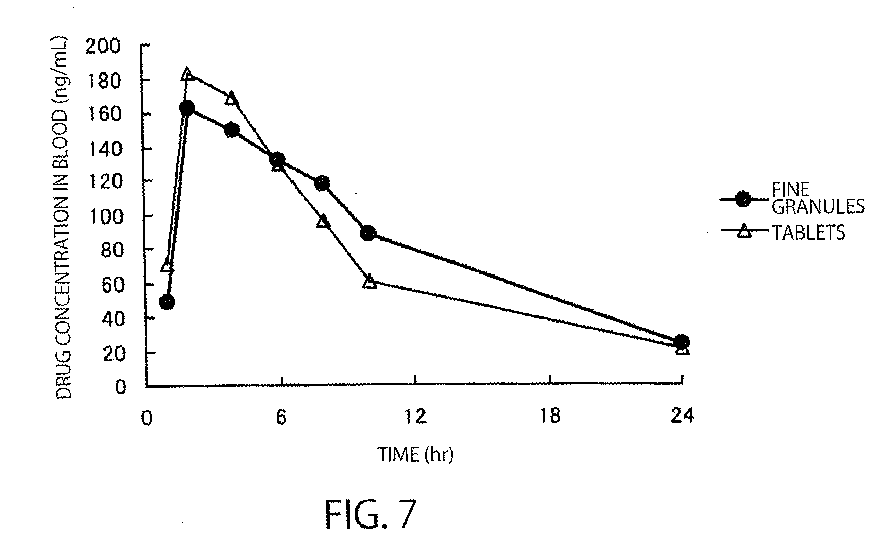

Oral absorbability in dogs (male beagle dogs, weight about 11 kg) was evaluated for the fine granules of Example 4 and the tablets of Example 6. The test was carried out by a crossover test using 10 dogs. Under Fed state, both the pharmaceutical preparations in an amount corresponding to 25 mg of 22β-methoxyolean-12-ene-3β, 24(4β)-diol bulk drug were taken with 10 mL of water. After the elapse of predetermined times (1 hr, 2 hr, 4 hr, 6 hr, 8 hr, 10 hr, and 24 hr), blood was collected, and the drug concentration in blood was measured with LC-MS/MS.
The results are shown in Fig. 7. Both the fine granules of Example 4 and the tablets of Example 6 had good oral absorbability.

Thus, the present invention can improve the dispersibility of 22β-methoxyolean-12-ene-3β, 24(4β)-diol, which is a slightly soluble drug, in water and the solubility thereof to ensure a high level of oral absorbability and, at the same time, can realize the persistence of physicochemical stability during the production process, whereby more homogeneous particles can be produced and high-quality pharmaceutical preparations can be provided.

## Claims

1. A composition comprising a slightly soluble drug and a hydrophilic surfactant.

2. The composition according to claim 1, wherein the slightly soluble drug is a triterpene derivative or a pharmaceutically acceptable salt thereof.

3. The composition according to claim 1, wherein the slightly soluble drug is 22β-methoxyolean-12-ene-3β, 24(4β)-diol or a pharmaceutically acceptable salt thereof.

4. The composition according to claim 1 or 2, wherein the hydrophilic surfactant is sodium laurylsulfate, a sucrose fatty acid ester, or polysorbate.

5. The composition according to claim 1 or 2, which further comprises a polymer compound.

6. The composition according to claim 5, wherein the polymer compound is a water soluble polymer.

7. The composition according to claim 6, wherein the water soluble polymer is polyvinyl pyrrolidone or hypromellose.

8. The composition according to claim 1 or 2, wherein the amount of the hydrophilic surfactant added is 10 to 400 mg per g of the triterpene derivative.

9. The composition according to claim 5, wherein the amount of the polymer compound added is 30 to 800 mg per g of the triterpene derivative.

10. The composition according to claim 3, wherein 22β-methoxyofean-12-ene-3β,24(4β)-diol has a cumulative 50% particle diameter of not more than 1.5 µm and a cumulative 90% particle diameter of not more than 3.0 µm.

11. The composition according to claim 3, wherein 22β-methoxyolean-12-ene-3β,24(4β)-diol has a cumulative 50% particle diameter of not more than 1.0 µm and a cumulative 90% particle diameter of not more than 2.0 µm.

12. A process for producing a composition according to any one of claims 1 to 3, **characterized by** comprising dispersing a slightly soluble drug and a hydrophilic surfactant in a solvent and then grinding the dispersion with a high-pressure homogenizer.

13. A process for producing a composition according to claim 5, **characterized by** comprising dispersing a slightly soluble drug and a hydrophilic surfactant in a solvent, grinding the dispersion with a high-pressure homogenizer, and then further dissolving a polymer compound in the homogenate.

14. The process according to claim 12 or 13, wherein the slightly soluble drug is 22β-methoxyolean-12-ene-3β, 24(4β)-diol or a pharmaceutically acceptable salt thereof.

15. A composition comprising a slightly soluble drug and a hydrophilic surfactant, the composition being producible by a process according to any one of claims 12 to 14.
